# EUROPÄISCHE PATENTSCHRIFT

(11) **EP 0 877 883 B1**
(45) Veröffentlichungstag und Bekanntmachung des Hinweises auf die Patenterteilung: **21.05.2003**
(21) Anmeldenummer: 97953685.1
(22) Anmeldetag: 17.11.1997
(51) Int. Cl.: F16K 31/00, A61M 39/22, A61M 5/168

(54) **MIKROVENTIL**
MICROVALVE
MICRO-SOUPAPE

(30) Priorität: 19.11.1996 DE 19647838; 10.12.1996 DE 19651285; 23.01.1997 DE 19702361
(43) Veröffentlichungstag der Anmeldung: 18.11.1998
(73) Patentinhaber: Lang, Volker, 82131 Gauting (DE)
(72) Erfinder: Lang, Volker, 82131 Gauting (DE)
(74) Vertreter: Laufhütte, Dieter, Dr.-Ing.
(86) Internationale Anmeldenummer: EP9706411
(87) Internationale Veröffentlichungsnummer: WO98022738

(56) Entgegenhaltungen:
- EP-A- 0 013 334
- EP-A- 0 189 491
- DE-A- 2 549 060
- US-A- 1 734 186
- US-A- 3 822 895
- US-A- 4 267 853
- US-A- 4 498 491

## Beschreibung

Die Erfindung betrifft ein Mikroventil für den Einmalgebrauch zur Verwendung in der analytischen Chemie oder der Medizintechnik sowie eine Verwendung eines derartigen Mikroventils.

Vor allem im Bereich der intravenösen Infusionstherapie und bei der Medikamentenapplikation, aber auch bei diagnostischen Maßnahmen, die mit Blutabnahmen verbunden sind, werden fast ausschließlich Einmalgebrauchsysteme aus hygienischen Gründen verwendet Alle hier eingesetzten aktiven Ventile werden noch ganz überwiegend manuell bedient und sind als Dreiwegehähne in Einzahl oder in Mehrzahl als Hahnenbatterien, ausgebildet. Daneben werden noch manuell bedienbare Schlauchklemmen als Ventile eingesetzt. Nur im Bereich von Herz-Lungen- und Dialysemaschinen werden aufwendige elektromechanische Ventile, fast ausschließlich jedoch als Schlauchquetschventile ausgebildet, verwendet. Wünschenswert wäre es deshalb, von ärztlicher, schwesterlicher wie auch hygienischer Seite aus betrachtet, diese arbeitsintensiven und bedienungsfehlerbehafteten Manipulationen auch in der Infusionstherapie und bei diagnostischen Maßnahmen durch eine Automatisierung der Ventilsteuerung zu ersetzen. Dies setzt aber die Einsatzmöglichkeit spezieller, sehr kostengünstiger, elektrisch steuerbarer Ventile voraus.

Aus der US 1,734,186 ist bereits eine Feuerlöscheinrichtung bekannt, bei der das Löschwasser mittels eines Ventils zurückgehalten wird, und welches aus einer Zelluloidschicht besteht. Diese Zelluloidschicht kann durch einen elektrisch aktivierbaren Heizdraht zerstört werden, so daß das Löschwasser nach entsprechender Aktivierung des Heizdrahts und Zerstörung der Zelluloidschicht austreten kann.

Aus der US 3,822,895 ist ein Air-Bag-System bekannt, bei dem das Gas zum Aufblasen des Air-Bags mittels eines Schmelzverschlusses in einer Druckkammer zurückgehalten wird.

Aufgabe der nachfolgend beschriebenen Erfindung war es, einfache, sichere, preiswerte, kleine, totraumarme, elektrisch auslösbare Ventile für die analytische Chemie aber vor allem für die Medizintechnik, hier besonders die Infusionstherapie, zu schaffen.

Erfindungsgemäß wird diese Aufgabe durch ein Mikroventil für den Einmalgebrauch mit den in Anspruch 1 definierten Merkmalen gelöst.

Erfindungsgemäß ist hier ein Mikroventil mit einfachen Mitteln geschaffen, das bei Bedarf durch Zufuhr elektrischer Energie einmal geöffnet bzw. geschlossen werden kann.

Bevorzugte Ausgestaltungen des erfindungsgemäßen Mikroventils ergeben sich aus den an den Hauptanspruch anschließenden Unteransprüchen. Besonders vorteilhaft ist die Verwendung eines erfindungsgemäßen Mikroventils in einer automatischen, programmierbaren Infusions-Medikamenten-Applikationsvorrichtung nach einem der Ansprüche 15 bis 20.

Einzelheiten und Vorteile der Erfindung werden anhand der in der Zeichnung dargestellten Ausführungsbeispiele gezeigt.
- Fig. 1: zeigt als Explosionszeichnung eine einfache Ausführungsform des elektrischen Einmalgebrauch-Mikroventils.
- Fig. 2: zeigt im Schnitt das elektrische Einmalgebrauch-Mikroventil von Fig. 1 (Schnittebene I....I).
- Fig. 3 und Fig. 4: zeigen schematisch die Funktion eines Einmalgebrauch-Mikroventils (Schließer) in Ruhe und nach elektrischer Betätigung.
- Fig. 5 und Fig. 6: zeigen schematisch die Funktion eines Einmalgebrauch-Mikroventils (Öffner) in Ruhe und nach elektrischer Betätigung.
- Fig. 7 und Fig. 8: zeigen als weiteres Beispiel die Funktion eines Einmalgebrauch-Mikroventils (Schließer), das einen Wachspfropfen mit offenem Zentralkanal in Kombination mit einem Siebplättchen als Verschlußelemente verwendet, in Ruhe und nach elektrischer Betätigung.
- Fig. 9 und Fig. 10: zeigen als weiteres Beispiel ein Mikroventil als Öffner, das nur einen einfachen Wachspfropfen als Schmelzverschluß verwendet, in Ruhe und nach elektrischer Betätigung.
- Fig. 11 und Fig. 12: zeigen als Beispiel ein Mikroventil als Öffner, das als Verschlußelement eine leicht schmelzbare Membran mit angepreßtem SMD-Widerstand oder Heizdraht verwendet, in Ruhe und nach elektrischer Betätigung.
- Fig. 13 und Fig. 14: zeigen schließlich als Beispiel noch ein Mikroventil als Öffner, bei dem das elektrische Widerstandselement direkt auf die leicht thermisch schmelzbare Ventilverschlußmembran zusammen mit den elektrischen Verbindungsleitungen aufgebracht ist.
- Fig. 15: zeigt als Anwendungsbeispiel aus der Medizintechnik für die Einmalgebrauch-Mikroventile eine programmierbare Infusions-Medikamenten-Applikationsvorrichtung in schematischer Darstellung.
- Fig. 16: zeigt perspektivisch die Infusions-Medikamenten-Applikationsvorrichtung mit den Teilen A für den Einmalgebrauch und B, C und D für den Mehrmalgebrauch.
- Fig. 17: zeigt perspektivisch Teil B, die Spritzenhaltevorrichtung mit Federantriebselementen.
- Fig. 18: zeigt perspektivisch Teil A die elektrisch gesteuerte Ventil-Dosier-Verteiler-Kassette mit Luerlockanschlüssen für Injektionsspritzen, Infusionsbehälter und Infüsionspumpen.
- Fig. 19: zeigt perspektivisch Teil E eine an Teil A zusätzlich anschließbare Spritzenhaltevorrichtung mit Federantriebselementen (nicht gezeichnet).
- Fig. 20 bis Fig. 26: zeigen perspektivisch die Einzelteile, aus denen Teil A zusammengefügt ist.

In Fig. 27 und Fig. 28 ist noch die aus Teil A herausgenommene aus zwei Teilen zusammengefügte Dosiervorrichtung dargestellt.

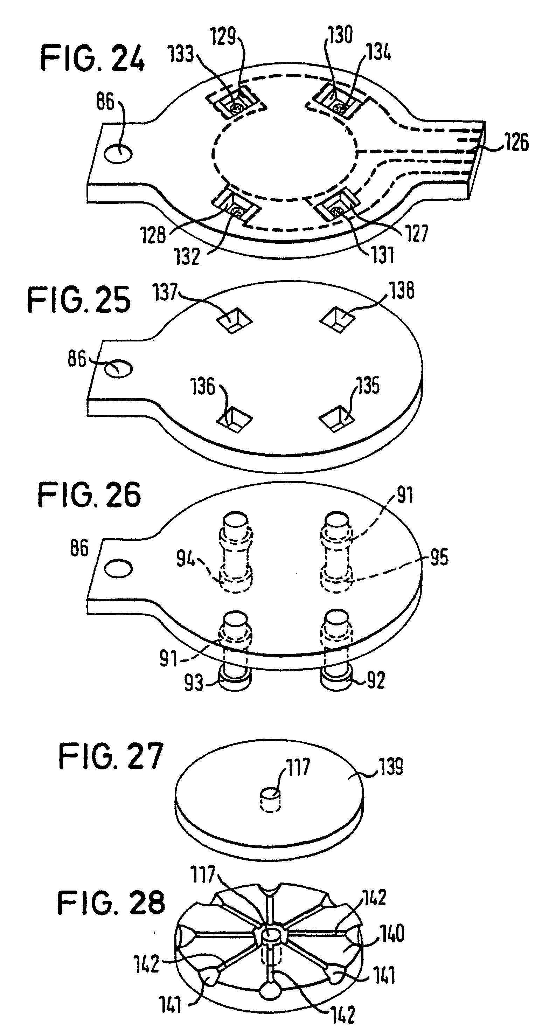

- Fig. 27: zeigt perspektivisch deren plane Deckplatte,
- Fig. 28: deren mit Dosierrillen versehene plane Bodenplatte.

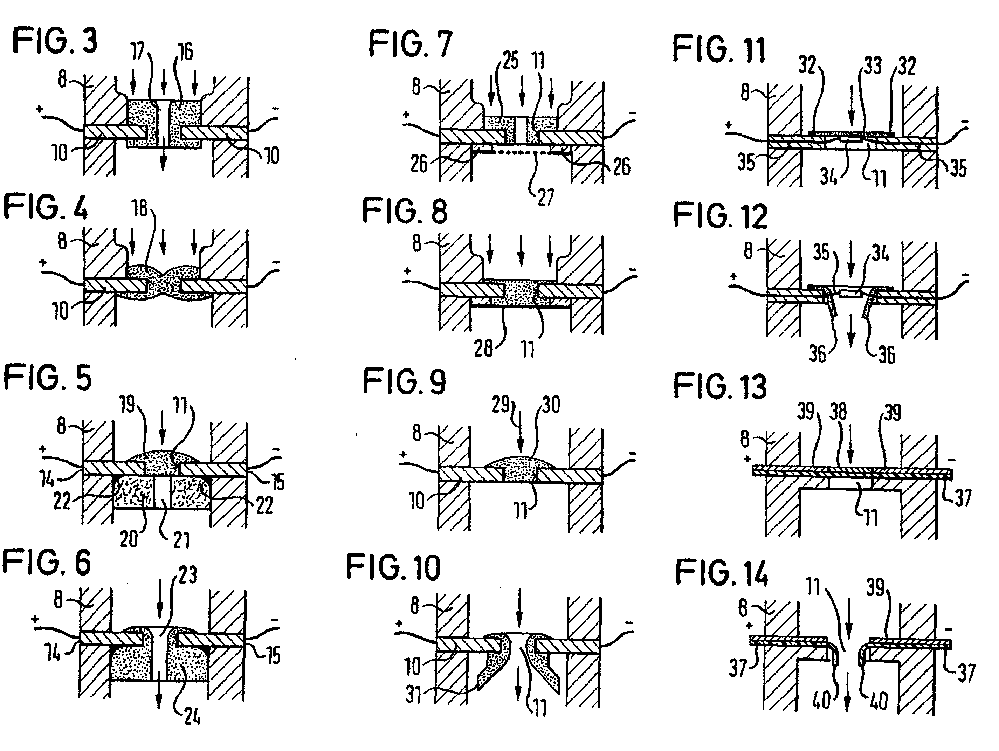

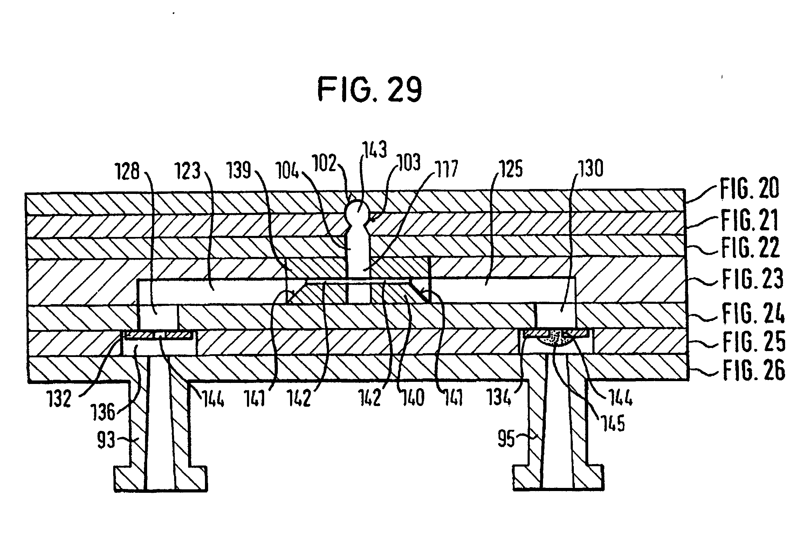

- Fig. 29: zeigt im Schnitt Teil A (Schnittebene I...I, Fig. 3).

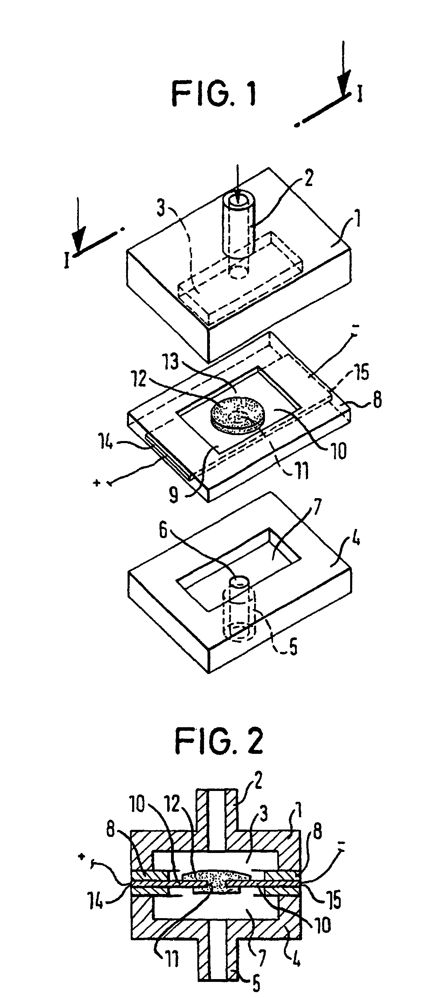

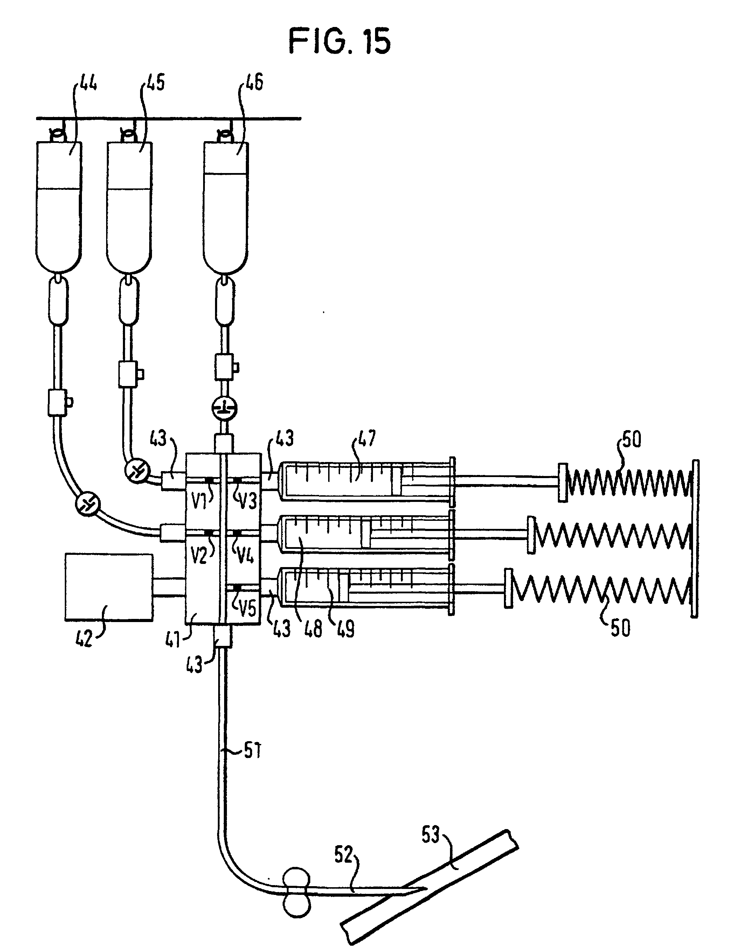

Die erfindungsgemäßen Ventile seien nachfolgend an Hand der Fig. 1-14 beschrieben und ihr Einsatz am Beispiel einer damit realisierten Infusions-Medikamenten-Applikations- Vorrichtung demonstriert (Fig. 15 - Fig. 29).

### Beschreibung der elektrischen Einmalgebrauch-Mikroventile:

Fig. 1 zeigt als Explosionszeichnung eine einfache Ausführungsform eines elektrischen Einmalgebrauch-Mikroventils mit Öffnerfunktion. Es ist aus einem durchsichtig gezeichneten Ventilgehäuseoberteil 1 mit Einlaßstutzen 2, Einlaßkammer 3 und einem Ventilgehäuseunterteil 4 mit Auslaßstutzen 5, Auslaßöffnung 6, sowie der Auslaßkammer 7, unter Zwischenschaltung der Ventilträgerplatte 8, dicht zusammengefügt. In die Ventilträgerplatte 8 mit ihrem rechteckigen Ausschnitt 9 ist ein gelochtes, rechteckiges PTC-Widerstandsplättchen in SMD-Technik 10 (PTC-Widerstand = elektrischer Widerstand mit positivem Temperaturkoeffizienten) gasdicht eingegossen bzw. eingeklebt und zwar so, daß dessen Loch bzw. Ventilöffnung 11 (gestrichelt gezeichnet, da durch den schmelzbaren Verschlußpfropfen 12 verdeckt) in einem breiten Saum 13 an der Ober- und Unterseite von Kunststoff bzw. Kleber unbedeckt bleibt. Damit wird aber auch das zusätzliche dichte Aufbringen von Verschlüssen und Sondermembranen problemlos möglich. Die noch notwendige elektrische Kontaktierung des PTC-plättchen kann sehr einfach über die Kontaktierungsstreifen 14, 15 vorgenommen werden.

Fig. 2 zeigt die Vorrichtung gemäß Fig. 1 zusammengebaut im Schnitt (Schnittebene I....I). Wie man sieht, ist der Ventilgehäuseoberteil 1, mit Einlaßstutzen 2 und Einlaßkammer 3, mit dem Ventilgehäuseunterteil 4 und dessen Auslaßstutzen 5, sowie Auslaßkammer 7, über die Ventilträgerplatte 8 fest und gasdicht verbunden. In den Rahmen der Ventilträgerplatte 8 ist das rechteckförmige PTC-Widerstandsplättchen 10 mit seinem Loch (Ventilöffnung) 11, das verschlossen ist durch den wärmeschmelzbaren Verschlußstopfen 12, gasdicht eingeklebt bzw. eingegossen. Die notwendige Stromzufuhr für das PTC-Widerstandsplättchen 10 erfolgt über an die Kontaktierungsstreifen 14 und 15 angelötete Kabel.

Die Fig. 3- 14 zeigen schematisch im Schnitt Ausführungsbeispiele der Mikroventile und ihre Funktion.

Die Fig. 3 stellt schematisch im Schnitt ein Ventil mit Schließerfunktion dar. Hier steckt im Loch (der Ventilöffnung) des PTC-Widerstandsplättchens 10 der wärmeschmelzbare Stopfen 16 mit seinem offenen Zentralkanal 17. In Fig. 4 ist der Zustand nach Ventilbetätigung dargestellt. Unter Einwirkung von elektrischem Strom erwärmt sich der PTC-Widerstand, das Material des Stopfens 16 schmilzt und verschließt als Stopfen 18 die Ventilöffnung.

Fig. 5 und Fig. 6 stellen ein Mikroventil mit Öffnerfunktion dar.

Im hier dargestellten Ausführungsbeispiel ist in Fig. 5 eine Ventilöffnung 11 in einem PTC-Widerstand durch einen wärmeschmelzbaren Stopfen 19 verschlossen. An der Unterseite des PTC-Widerstandes ist ein nur an seinen Rändern 22 angeklebtes, mit zentralem Loch 21 versehenes, dickes Plättchen 20 aus saugfähigem Material (z.B. Zellulosefasern) angeordnet. Wie man in Fig. 6 deutlich sieht, kommt es unter Stromeinwirkung zur Erwärmung mit Schmelzen des Verschlußstopfens 19 und Aufsaugen des verflüssigten Materials 24 unter Eröffnung des Kanals 23.

Fig. 7 und Fig. 8 stellen eine geeignete Mikroventilkonstruktion mit Schließfunktion bei höherer Druckbelastung dar. Hier steckt in der Ventilöffnung 11 des PTC-Widerstandplättchens 10 ein wärmeschmelzbarer Stopfen mit offenem Zentralkanal 25. An der Unterseite des PTC-Widerstandplättchens ist zusätzlich ein dünnes Sieb- (Maschen-, Löcher-, Poren-, Flies-) Plättchen 27 aus Metall oder Kunststoff angebracht (geklebt). Nach Erhitzung unter Stromeinwirkung sieht man in Fig. 8 die Ventilöffnung 11, ebenso wie die feinen Löcher des Siebplättchens durch wärmeschmelzbares Material verschlossen. Ein weiteres Ausführungsbeispiel (Fig. 9 und 10) zeigt wie die unter Druck 29 stehende nur durch einen Wachsstopfen 30 verschlossene Ventilöffnung 11 nach Erwärmung des PTC-Widerstandes unter Stromeinwirkung zu einer Verflüssigung des Stopfens mit Freiblasen der Ventilöffnung von Wachs 31, s. Fig. 10, führt.

Fig. 11 und Fig. 12 zeigen noch eine Realisierung von Mikroventilen durch aufgeklebte (Kleberand 32), wärmeschmelzbare Kunststoffolien oder Wachsmembranen 33, welche die Ventilöffnung 11 gasdicht verschließen und auf die im Bereich der Ventilöffnung 11 ein elektrisch aufheizbarer Widerstand 34 (z.B. SMD-Widerstand, Widerstandsdraht) einwirkt.

Fig. 12 zeigt nach Stromeinwirkung den Widerstand 34 an seinen Drahtenden 35 hängend in der Ventilöffnung 11 und die Reste der eröffneten Ventilmembran 36. Fig. 13 und Fig. 14 zeigen schließlich noch eine Mikroventilausführung (Öffner), bei der das elektrische Widerstandselement 38 direkt auf der leicht schmelzbaren Ventilverschlußmembran 37 zusammen mit den elektrischen Verbindungsleitungen 39 aufgebracht ist (z.B. durch Aufdampfen oder Aufdrucken). Fig. 14 zeigt den Zustand nach Ventilbetätigung. Die Verschlußfolie 37 ist im Bereich der Ventilöffnung 11 aufgerissen und zeigt aufgeklappte Reste 40 derselben.

Fig. 15 zeigt noch schematisch als Anwendungsbeispiel für diese Mikroventile aus dem Bereich der Medizintechnik, eine programmierbare Infusions-Medikamenten-Applikationsvorrichtung, die für die ambulante wie auch stationäre Patientenbehandlung konzipiert ist. Das Herzstück dieser Applikationsvorrichtung stellt eine über die programmierbare, elektronische Steuer-Stromversorungs-Vorrichtung 42 angeschlossene Einmalgebrauchskassette 41 dar. An diese sind über Luerlockanschlüsse 43, sowohl Schwerkraftinfusionsvorrichtungen 44, 45, 46, jeweils bestehend aus Infusionsbehälter, Tropfkammer und Ableitsystem mit Tropfregler und Einwegventil, als auch unter Federdruck 50 stehende Injektionsspritzen (Medikamenten-Vorratsbehälter) 47, 48, 49 (mit Ausnahme von Vorrichtung 46) über die eingebauten elektrischen Mikroventile (V1 bis V5), angeschlossen. Über die Schwerkraftinfusionsvorrichtung 46 werden die Kassette 41, die daran angeschlossene Patienten-Infusionsleitung 51 und die in der Patientenvene 53 fixierte Butterfly-Hohlnadel 52 langsam, kontinuierlich mit Infusionslösung durchströmt. Durch Öffnen der Mikroventile V1 bis V5 nach individuell vorgewähltem Programm durch die elektronische Steuer-Strom-Versorgungsvorrichtung 42, können nun noch vollautomatisch die gewünschten Infusionen 44, 45 und Medikamente 47 bis 49 an den Patienten verabreicht werden. Selbstverständlich lassen sich die beschriebenen kostengünstigen Einmalgebrauch-Mikroventile z.B. vorteilhaft auch bei transportablen chemischen Mikroanalysegeräten einsetzen.

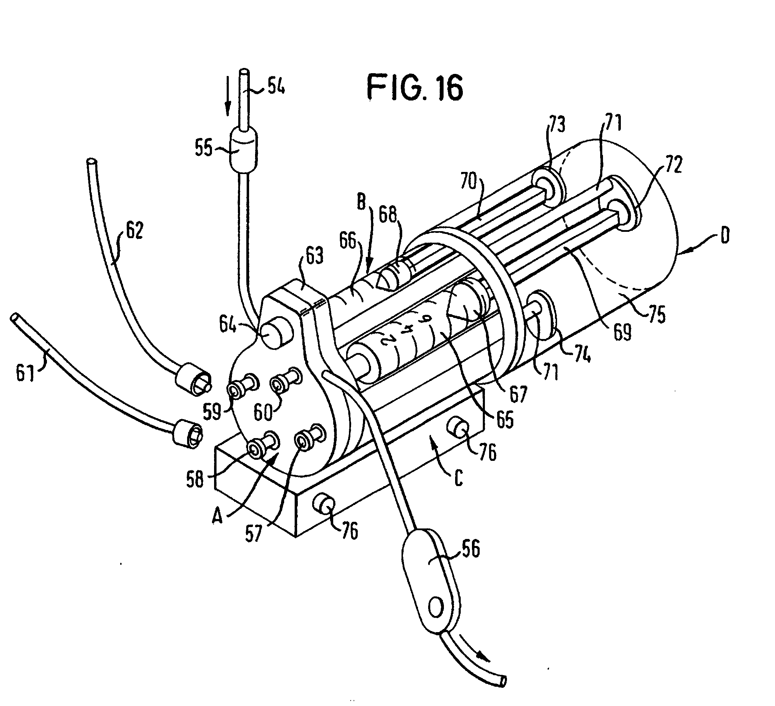

An Hand der Fig. 16 - Fig. 29 erfolgt noch nachfolgend die Beschreibung der konstruktiv realisierten Infusions-Medikamenten-Applikations-Vorrichtung von Fig. 15.

### Beschreibung der Infusions-Medikamenten-Applikationsvorrichtung:

Wie in Fig. 16 dargestellt, besteht sie aus den Teilen A, B, C und D. Teil A, der Ventil-Dosier-Verteiler-Kassette, wird über einen Infusionsschlauch 54 mit eingebautem Einwegventil 55, wie durch einen Pfeil angedeutet, sterile Infusionsfiüssigkeit auf konventionelle Art, z.B. aus angehängten Flaschen oder Beuteln unter Ausnutzung der Schwerkraft, zugeführt und über den abführenden Schlauch mit nachgeschaltetem Bakterien-Entlüftungsfilter 56 an den angeschlossenen Patienten (in Pfeilrichtung) abgegeben. Über die Luerlock-Konnektoren mit nachgeschalteten Partikelfiltern 57, 58, 59, 60 können zusätzlich noch über Infusionsleitungen mit Lueriock-Konnektoren 61, 62 Medikamentenlösungen aus unter Druck stehenden Behältern, z.B. Beuteln oder Spritzen, zusätzlich in Teil A eingespeist werden. Wie man in Fig. 16 sieht ist Teil B, die Spritzenhaltevorrichtung mit Federantriebselementen mit Hilfe ihrer Anschlußplatte 63 an Teil A angeflanscht und mit Verriegelungselement 64 gesichert. Die zwei in den Halterungen eingeschobenen 10 ml Spritzen 65, 66 sind mit ihren Luerlockanschlüssen (im Bild nicht sichtbar) mit zwei der an Teil A an dieser Seite zusätzlich noch angebrachten vier Luerlockanschlüssen verriegelt. Ihre Spritzenkolben 67, 68 mit den zugehörigen Spritzenstempeln 69, 70 werden über drehbar an Zugstangen 71 angebrachten Druckplatten 72, 73 in die Spritzenzylinder hineingeschoben. Die gezeichnete Zugstange 71 mit zugehöriger Druckplatte 74 soll noch die Ruhestellung des eingebauten Federantriebelements demonstrieren. Als Berührungsschutz für Antriebselemente und Spritzenstössel dient der auf Teil B verriegelte, durchsichtige, zylindrische Schutzdeckel 75 (Teil D).

Die an der Vorder- und Rückseite von Teil C, der programmierbaren, elektrisch-elektronischen Steuer-Stromversorgungseinheit, angebrachten Gewindebolzen 76, können als Befestigungselemente für Aufhängebügel dienen.

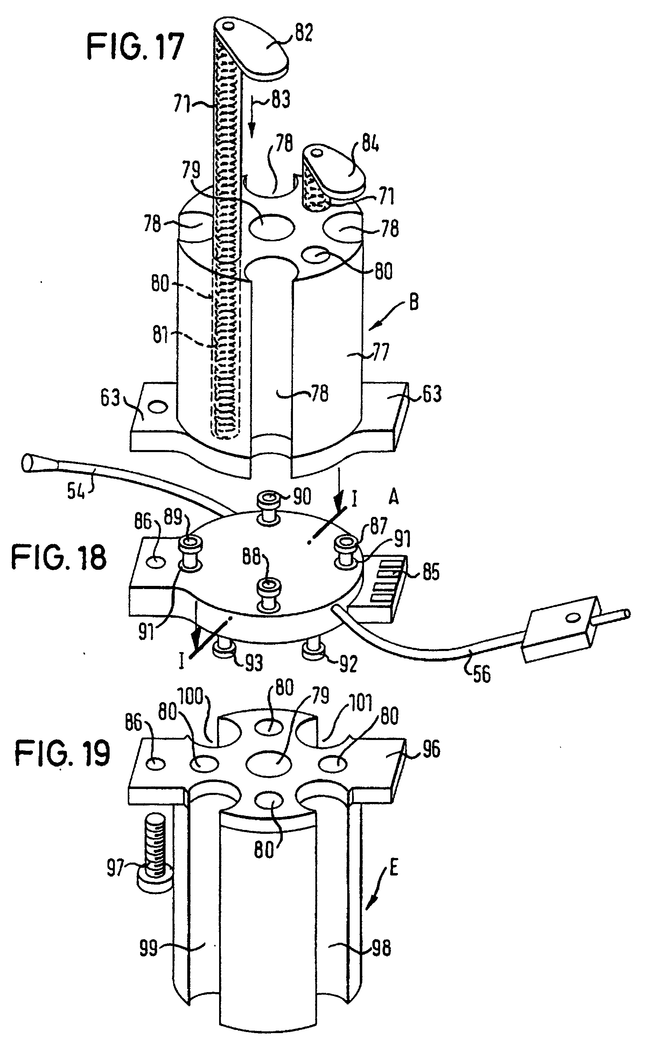

Fig. 17 zeigt perspektivisch Teil B die Spritzenhaltevorrichtung mit integrierten Federantriebselementen.

Wie man deutlich sieht besteht die Spritzenhaltevorrichtung aus einem zylindrischen Körper 77 mit angeflanschter Anschlußplatte 63, der vorzugsweise aus leichtem Kunststoff gefertigt ist. In diesen zylindrischen Körper sind 4 zylindrische Längsnuten 78, als Halterung für die Spritzen, eine große zentrale Bohrung 79 zur Gewichtsreduktion und 4 Bohrungen 80 zur Aufnahme der Federantriebselemente eingebracht. Der Übersicht halber sind nur zwei Federantriebe (in gespanntem und entspanntem Zustand) gezeichnet. Der vereinfacht, links im Bild, gespannt dargestellte Federantrieb besteht aus der am Boden der Bohrung 80 verankerten maximal gespannten Zugfeder 81, die in ihrem oberen Teil von dem Führungsrohr = Zugstange 71, die schwenkbar die Druckplatte 82 trägt, umschlossen ist. Diese Druckplatte schiebt den anliegenden Spritzenstempel und Kolben der eingelegten Spritze in eingezeichneter Pfeilrichtung 83. Rechts im Bild ist der Federantrieb in entspannter Position (Ruhestellung) mit seiner Druckplatte 84 dargestellt.

Fig. 18 zeigt perspektivisch Teil A, die Ventil-Dosier-Verteiler-Kassette mit Elektroanschlußstecker 85, Verriegelungsloch 86, den Anschlußschläuchen 54 und 56, sowie Luerlockanschlüssen 87, 88, 89, 90 sind jeweils Partikelfilter 91 nachgeschaltet, welche die Dosierkapillaren des nachgeschalteten, eingebauten Dosierverteilers vor Obstruktion schützen. An der Unterseite von Teil A sind ebenfalls 4 Luerlockanschlüse 92, 93, 94, 95 (sichtbar nur 92, 93), jeweils um 45 Grad zu denen der Oberseite verdreht, angebracht. Jeweils sind auch diesen Luerlockanschlüssen Partikelfilter 91 nachgeschaltet.

Fig. 19 zeigt als mögliches Ergänzungsteil E eine zweite Spritzenhaltevorrichtung mit Anschlußplatte 96 und Verriegelungselement 97, jedoch noch nicht mit Federantriebselementen bestückt. Deutlich sieht man auch wie bei einem Zusammenbau jeweils die Luerlockanschlüsse 92 und 93 und die nicht sichtbaren Anschlüsse 94 und 95 in die Spritzenhaltenuten 98 und 99 bzw. 100 und 101 zu liegen kommen.

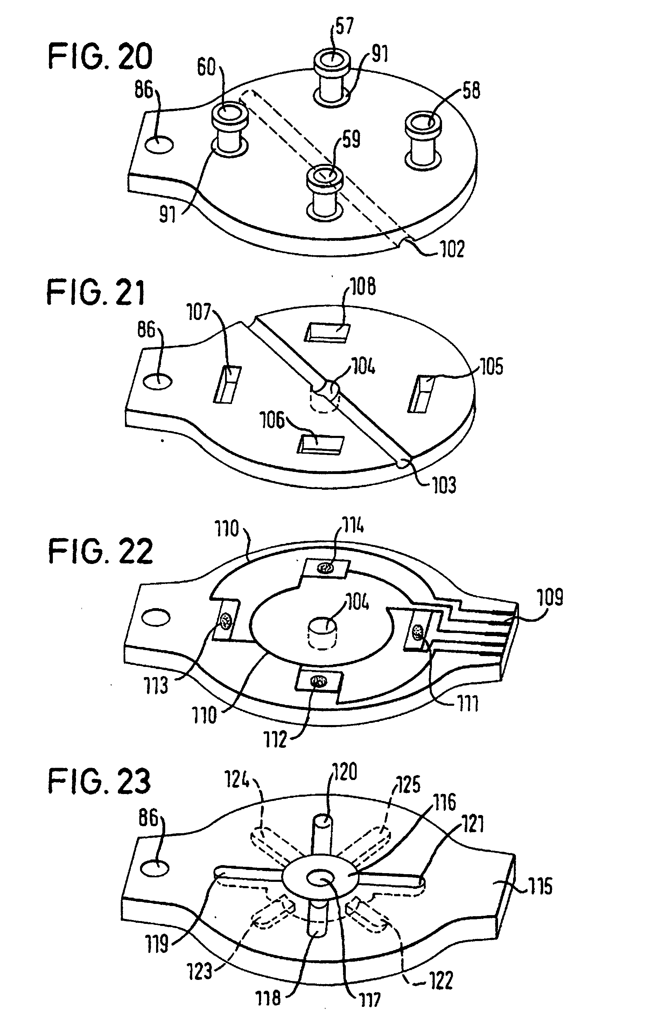

Fig. 20 - 26 zeigen perspektivisch, aufeinanderfolgend die Einzelteile, aus denen Teil A zusammengefügt ist.

Fig. 20 zeigt die runde Deckplatte mit Fortsatz von Teil A mit Verriegelungsloch 86, dem Oberteil des Kanals 102 und den 4 Luerlockanschlüssen 75, 58, 59, 60, denen jeweils Partikelfilter 91 nachgeschaltet sind.
Fig. 21 die nachfolgende runde Platte mit Fortsatz, Verriegelungsloch 86, dem Unterteil des Kanals 103 mit zentralem Kanal 104, den 4 rechteckigen Ausschnitten 105, 106, 107, 108 für die in Fig. 7 dargestellten Mikroventile.

Fig. 22 stellt die erste Ventilplatte dar mit dem Verriegelungsfortsatz mit Loch 86 und dem durch aufgedruckte, elektrische Kontakte zum Elektrostecker umgewandelten Fortsatz 109. Daneben weist diese Platte einen zentralen Kanal 104, vier rechteckige, mit elektrischen Mikroventilen 111, 112, 113, 114 dicht abgedeckte Ausschnitte und aufgedruckte, die Ventile verbindende, elektrische Leiterbahnen 110, auf.

Fig. 23 zeigt die Mikrodosier-Verteilerplatte. Sie weist zwei Fortsätze auf, den einen mit Verriegelungsloch 86 versehen, den anderen als Widerlager 115 ausgebildet für den Elektrostecker 109 (siehe Fig. 7). Im Zentrum der Scheibe befindet sich die Dosiervorrichtung 116 mit Zentralkanal 117 und ihren 2 x 4, jeweils in zwei Ebenen und um 45 Grad gegeneinander verdrehten und zur Oberseite bzw. Unterseite jeweils offenen Zubringerkanälen 118, 119, 120, 121 bzw. 122, 123, 124, 125.

Fig. 24 stellt die zweite Ventilplatte dar mit dem Verriegelungsfortsatz mit Loch 86 und dem, durch die auf der Unterseite (gestrichelt dargestellt) aufgedruckten, elektrischen Kontakte zum Elektrostecker umgewandelten Fortsatz 126. Daneben weist diese Platte vier rechteckige Öffnungen 127-130 auf, deren Boden jeweils durch vier dicht aufgebrachte Mikroventile 131-134 gebildet wird. Diese Mikroventile sind durch auf der Unterseite der Platte aufgebrachte, gedruckte Leiterbahnen (gestrichelt gezeichnet) sowohl untereinander, als auch mit dem Elektrostecker 126 verbunden.

Fig. 25 zeigt eine runde Platte mit Fortsatz und Verriegelungsloch 86 und vier rechteckigen Ausschnitten 135-138 für die vier Mikroventile 131-134.

Fig. 26 zeigt wiederum eine runde Platte mit Fortsatz und Verriegelungsloch 86, die auf ihrer Unterseite vier Luerlockanschlüsse 92, 93, 94, 95 mit jeweils nachgeschalteten Partikelfiltern 91 aufweist.

Fig. 27 stellt die plane Deckplatte 139 der Dosiervorrichtung mit zentralem Kanal 117 dar und Fig. 28 die dazugehörige plane Bodenplatte 140 mit den acht in den Zentralkanal 117 einmündenden, an ihrem Anfang 141 jeweils erweiterten Dosierrillen 142. Durch das Zusammenfügen dieser beiden Platten 139 und 140 entsteht die Dosiervorrichtung 116.

Fig. 29 zeigt im Schnitt Teil A (Schnittebene I...I, siehe Fig. 3). Wie man sieht, ist Teil A aus sieben Schichten zusammengefügt entsprechend den Darstellungen in Fig. 20 bis Fig. 26. Entsprechend der Schnittebene I...I finden sich in den oberen zwei Schichten, siehe Fig. 20 und Fig. 21, das Oberteil 102 und das Unterteil 103 des Kanals 143, in dessen Enden Infusionsschlauch 54 bzw. abführender Schlauch 56 eingeklebt sind und der über den Kanal 104 (siehe Fig. 21 und Fig. 22) mit dem Zentralkanal 117, der Dosiervorrichtung, welche aus der Deckplatte 139 und der Bodenplatte 140 gebildet wird, verbunden ist.

Wie man sieht, erhält diese Dosiervorrichtung über den Luerlockanschluß 93, einen durch den Ausschnitt 136 gebildeten Ventilvorraum, das Mikroventil 132 mit seiner Öffnung 144, einen durch den Ausschnitt 128 gebildeten nachgeschalteten Raum und den Verteilerkanal 123 Infusions-Medikamentenlösung geliefert. Diese fließt durch das erweiterte Anfangsstück 141 der Dosierrinne (Kapillare) 142 zum Zentralkanal 117 und von dort, wie geschildert, 104, 143 schließlich in den abführenden Schlauch 56. Auch über Luerlockanschluß 95 kann Infusionslösung, wenn das verschlossene (Verschluß 145) Mikroventil 134 elektrisch geöffnet wird, über seine Ventilöffnung 144, den durch den Ausschnitt 130 gebildeten nachgeschalteten Raum und Verteilerkanal 125 zur Dosiervorrichtung und deren zugehöriger Dosierkapillare 142 fließen und von dort über deren Zentralkanal 117 schließlich weiter in den Kanal 143 und abführenden Schlauch 56.

### Funktion der Infusions-Medikamenten-Applikationsvorrichtung:

Wie Fig. 16 zeigt, wird Teil A, die Einmalgebrauchs-Kassette, nach Entnahme aus ihrer Sterilverpackung mit ihrem Elektrostecker 126 in die programmierbare SteuerStrom-Versorgungseinheit C (nach Stand der Technik) eingesteckt. Als Beispiel, sollen bei einem mit venösem Katheter versehenen Patienten, vier Medikamente, zu unterschiedlichen, exakt definierten Zeiten, neben zwei Kurzinfusionen und einer Dauerinfusion, verabfolgt werden. Die Realisierung dieser Therapie erfordert nach dem heutigen Standard der Krankenhäuser schon zwei programmierbare Infusionspumpen für die Kurzinfusionen neben einem Arzt oder einer Schwester, welche die Medikamente zu den gewünschten Zeiten noch von Hand spritzen müssen.

Bei Verwendung der neuen Infusions-Medikamenten-Applikationsvorrichtung muß man nur Mehrmalgebrauchsteil B (siehe Fig. 1) mit Teil A verbinden, die vier gelösten Medikamente in handelsübliche 10 ml Spritzen, möglichst ohne Luft aufziehen und dann jeweils die Spritzen nach dem Einschieben in die nutenförmigen Halterungen von Teil B mit den Luerlockanschlüssen von Teil A verriegeln. Nun werden jeweils die Zugstangen 71 mit Druckplatten 72, 74 für die Spritzenstempel manuell herausgezogen, damit die Zugfedern 81 gespannt_und der Spritzeninhalt unter Druck gesetzt. Ebenso einfach können z.B. unter Feder- oder hydrostatischem Druck stehende Kleinbehälter für Kurzinfusionen über Einwegventile an Teil A (Luerlockanschlüsse 57-60) angeschlossen werden. Die Dauerinfusion kann über Anschluß 54, die Verbindung zum Patienten über Anschluß 56 (mit Bakterien-Entlüftungsfilter) vorgenommen werden. Die Vorprogrammierung von Teil C, der elektrisch-elektronischen Steuer-Stromversorgungseinheit (Kleinakkubetrieb), kann sehr einfach mit einem über eine Infrarot-Schnittstelle angeschlossenen üblichen Personalcomputer, vor oder auch noch nach Beginn der Infusionstherapie, mit Hilfe eines einfachen Programms vorgenommen und auch dokumentiert werden.

Kurzinfusionen, wie auch Medikamentenapplikationen, erfolgen nun vollautomatisch zu den gewünschten Zeiten durch jeweiliges Öffnen der zugehörigen Mikroventile und Dosiervorrichtungen der Einmalgebrauch-Kassette (Teil A). Durch Zusatzteil E können bei Bedarf auch noch weitere vier Medikamentenspritzen angeschlossen werden. Ebenso einfach läßt sich bei der ärztlichen Vorprogrammierung eine evtl. gewünschte, vom Patienten steuerbare, zusätzliche oder vorzeitige Medikamentenabgabe ins Infusionssystem, realisieren. Der in Fig. 16 dargestellte, verriegelbare Schutzdeckel D ermöglicht darüber hinaus noch einen weitgehenden Schutz vor Medikamentenmißbrauch.

## Patentansprüche

1. Mikroventil für den Einmalgebrauch zur Verwendung in der Medizintechnik oder der analytischen Chemie mit einem Gehäuse mit mindestens einem Ventilsitz und einem unter Wärmezufuhr schmelzendem Verschlußelement zum Öffnen oder Schließen des Mikroventils und mit elektrischen Heizelementen, die der Wärmezufuhr dienen, wobei aufgrund des Schmelzens des schmelzenden Verschlußelements eine zentrale Öffnung des Verschlußelements zur Öffnung des Mikroventils aufschmilzt oder wobei das Verschlußelement beim Schmelzen die zentrale Öffnung schließt, um das Mikroventil zu schließen.

2. Mikroventil nach Anspruch 1, **dadurch gekennzeichnet, daß** am Ventilsitz und/oder Verschlußelement zusätzliche Sonderdiaphragmen angeordnet sind.

3. Mikroventil nach Anspruch 1 oder 2, **dadurch gekennzeichnet, daß** die unter Wärmezufuhr schmelzenden Verschlußelemente Folien, Plättchen und/oder Stopfen sind, die aus leicht schmelzbaren Kunststoffen oder Wachsen hergestellt sind und gegebenenfalls einen zentralen Kanal aufweisen können.

4. Mikroventil nach einem der Ansprüche 1 bis 3, **dadurch gekennzeichnet, daß** das elektrische Heizelement als Ohmscher Widerstand, der gegebenenfalls aufgedruckt oder aufgedampft sein kann, PTC-Widerstand und/oder Heizdraht ausgebildet ist und fest auf die Verschlußelemente aufgepreßt oder direkt auf diese aufgebracht ist.

5. Mikroventil nach Anspruch 1 oder 2, **dadurch gekennzeichnet, daß** die Verschlußelemente aus elektrisch leitendem Material bestehen.

6. Mikroventil nach einem der Ansprüche 1 bis 5, **dadurch gekennzeichnet, daß** als elektrische Heizelemente gelochte Widerstandsplättchen, vorzugsweise in SMD-Technik, dienen, wobei deren Ventilöffnungen durch aufgepreßte, verklebte und/oder verschweißte unter Wärmezufuhr schmelzende Verschlußelemente verschlossen sind.

7. Mikroventil nach einem der Ansprüche 1 bis 4, **dadurch gekennzeichnet, daß** die elektrischen Heizelemente gelochte PTC-Widerstände, vorzugsweise in Plättchenform, Scheibchenform und SMD-Technik darstellen, wobei diese thermostatisierte Heizelemente und Ventilsitze mit Ventilöffnungen darstellen.

8. Mikroventil nach Anspruch 2, **dadurch gekennzeichnet, daß** die Sonderdiaphragmen aus einem Faserfilz, beispielsweise aus Zellulose bestehen, die feine Kapillaren enthalten.

9. Mikroventil nach Anspruch 2, **dadurch gekennzeichnet, daß** die Sonderdiaphragmen als Sieb, Loch, Gitter oder Porenplättchen ausgebildet sind und aus Metall oder Kunststoff bestehen.

10. Mikroventil nach einem der vorangehenden Ansprüche, **dadurch gekennzeichnet, daß** die elektrischen Heizelemente mit Ventilsitz und Ventilöffnung, gelochte SMD-Widerstände oder PTC-Widerstände, mit ihrem aufgebrachten wärmeschmelzbaren Verschlußelementen so in Gehäuse mit Ein- und Auslaßkanälen eingeklebt bzw. eingegossen sind, daß sie als gasdichte Scheidewände Ein- und Auslaßkanäle trennen.

11. Mikroventil nach einem der vorangehenden Ansprüche, **dadurch gekennzeichnet, daß** die elektrischen Heizelemente durch einen so starken Stromstoß aktivierbar sind, daß es bei Flüssigkeitseinsatz im Bereich der Heizelemente zur Dampfblasenbildung kommt

12. Mikroventil nach einem der vorangehenden Ansprüche, **dadurch gekennzeichnet, daß** den elektrischen Heizelementen mit Verschlußelement jeweils eine luftgefüllte Kammer vor- und/oder nachgeschaltet ist.

13. Mikroventil nach einem der vorangehenden Ansprüche, **dadurch gekennzeichnet, daß** das Verschlußelement eine Verschlußfolie ist, auf die die elektrischen Heizelemente sowie die dazugehörigen elektrischen Verbindungsleitungen und Anschlußstecker unmittelbar aufgebracht sind, beispielsweise aufgedruckt und/oder aufgedampft sind.

14. Mikroventil nach einem der vorangehenden Ansprüche, **dadurch gekennzeichnet, daß** die elektrischen Heizelemente beim Ventilauslösevorgang elektrisch so hoch belastbar sind, daß ihre Widerstandsstruktur zerstörbar, d.h. unterbrechbar ist und daß dieser Vorgang zur elektrischen Signalauslösung für eine adäquate Ventilfunktion nutzbar ist.

15. Verwendung eines Mikroventils nach einem der Ansprüche 1 bis 14 in einer Infusions-Medikamenten-Applikationsvorrichtung, wobei eine Einmal-Gebrauchskassette mit Mikroventilen, Verteilern, Luerlockanschlüssen und Dosiervorrichtungen genutzt wird, an die unter Druck stehende herkömmliche Pumpen-, Druck- oder Schwerkraftinfusionssysteme über Einwegventile in Kombination mit Medikamentenbehältern, die als federbelastete Injektionsspritzen ausgebildet sind, angeschlossen sind und bei der diese Mikroventile durch eine angesteckte, externe, programmierbare Steuer-Strom-Versorgungseinheit zu vorprogrammierten Zeiten zur Entleerung ihres Inhalts in die Infusionswege zum angeschlossenen Patienten geöffnet werden können.

16. Verwendung nach Anspruch 15, **dadurch gekennzeichnet, daß** die Anordnung der Luerlockanschlüsse, Mikroventile, Verteiler und eventuell zusätzlich noch verwendeter Dosierkapillaren zur Baugrößen- und Totraumminimierung vorzugsweise sternförmig ist.

17. Verwendung nach Anspruch 15 oder 16, **dadurch gekennzeichnet, daß** die federbelasteten Injektionsspritzen in einer zylinderförmigen Halterung, welche 4- oder mehr zylinderförmige Spritzen mit jeweils dazugehörigen Spritznuten mit jeweils dazugehörigen Zugfederelementen aufweist, angeordnet sind.

18. Verwendung nach einem der Ansprüche 17, **dadurch gekennzeichnet, daß** ein Zugfederelement aus einem Zugstangenrohr mit angelenkter Spritzen-Stempeldruckplatte und mit innenliegender Spiralzugfeder besteht, das in einer Büchse der zylinderförmigen Halterung gleitet.

19. Verwendung nach einem der Ansprüche 15 bis 18, **dadurch gekennzeichnet, daß** eine Dosiervorrichtung aus einer runden, planen Bodenplatte, in die sternförmige Präzisionsrillen eingebracht sind, besteht, die in einer gemeinsamen zentralen Bohrung münden und einer darauf geklebten planen, runden Deckplatte, die ebenfalls eine solche zentrale Bohrung aufweist, besteht.

20. Verwendung nach einem der Ansprüche 15 bis 19, **dadurch gekennzeichnet, daß** die Dosiervorrichtung jeweils auf der Bodenplatte und der Deckplatte eine Nut zur Aufnahme eines O-Dichtungsrings aufweist.

## Claims

1. Disposable microvalve for use in medical technology or analytical chemistry, with a housing with at least one valve seat and a closure element which melts under application of heat in order to open or close the microvalve, and with electrical heating elements for applying the heat, where, as a result of the fusion of the melting closure element, a central opening of the closure element melts open so as to open the microvalve, or where the closure element, upon melting, closes the central opening so as to close the microvalve.

2. Microvalve according to Claim 1, **characterized in that** additional special membranes are arranged on the valve seat and/or closure element.

3. Microvalve according to Claim 1 or 2, **characterized in that** the closure elements which melt under application of heat are films, plates and/or plugs which are made of easily fusible plastics or waxes and may optionally have a central channel.

4. Microvalve according to one of Claims 1 to 3, **characterized in that** the electrical heating element is designed as an active resistor, which can optionally be printed-on or vapour-deposited, a PTC resistor and/or a resistance wire, and is pressed firmly onto the closure elements or applied directly to these.

5. Microvalve according to Claim 1 or 2, **characterized in that** the closure elements are made of electrically conductive material.

6. Microvalve according to one of Claims 1 to 5, **characterized in that** the electrical heating elements used are perforated resistor plates, preferably in SMD technology, their valve openings being closed off by closure elements which are pressed on, glued on and/or welded on and melt under application of heat.

7. Microvalve according to one of Claims 1 to 4, **characterized in that** the electrical heating elements are perforated PTC resistors, preferably in plate form, disc form and SMD technology, these representing thermostatically controlled heating elements and valve seats with valve openings.

8. Microvalve according to Claim 2, **characterized in that** the special membranes are made of a fibre felt, for example of cellulose, containing fine capillaries.

9. Microvalve according to Claim 2, **characterized in that** the special membranes are designed as a sieve, perforation, grating or porous plate and are made of metal or plastic.

10. Microvalve according to one of the preceding claims, **characterized in that** the electrical heating elements with valve seat and valve opening, perforated SMD resistors or PTC resistors, with their attached hot-melt closure elements, are glued or cast into housings with inlet and outlet channels in such a way that they separate inlet and outlet channels in the manner of gas-tight partition walls.

11. Microvalve according to one of the preceding claims, **characterized in that** the electrical heating elements can be activated by such a high current impulse that vapour bubbles are formed in the area of the heating elements when liquid is used.

12. Microvalve according to one of the preceding claims, **characterized in that** an air-filled chamber is arranged upstream and/or downstream of each of the electrical heating elements with closure element.

13. Microvalve according to one of the preceding claims, **characterized in that** the closure element is a closure film onto which the electrical heating elements and the associated electrical connection leads and attachment plugs are directly applied, for example printed-on and/or vapour-deposited.

14. Microvalve according to one of the preceding claims, **characterized in that** the electrical heating elements can be subjected to such a high electrical load, during the valve-triggering procedure, that their resistance structure can be destroyed, i.e. interrupted, and that this procedure for electrical signal-triggering can be utilized for an adequate valve function.

15. Use of a microvalve according to one of Claims 1 to 14 in an apparatus for administering infusion medicaments, use being made of a disposable cartridge with microvalves, distributors, Luer lock attachments and metering devices to which pressurized conventional pump infusion, pressure infusion or gravity infusion systems are attached via one-way valves in combination with medicament containers designed as spring-loaded injection syringes, and in which these microvalves can be opened by an attached, external and programmable control current supply unit at pre-programmed times in order to empty its content into the infusion lines to the connected-up patient.

16. Use according to Claim 15, **characterized in that** the arrangement of the Luer lock attachments, microvalves, distributors and, optionally, additionally used metering capillaries is preferably star-shaped in order to minimize the structural size and dead space.

17. Use according to Claim 15 or 16, **characterized in that** the spring-loaded injection syringes are arranged in a cylindrical holder which has 4 or more cylindrical syringes, each with associated syringe grooves with associated tension spring elements.

18. Use according to one of Claims 17, **characterized in that** a tension spring element consists of a drawbar with articulated syringe plunger pressure plate and with internal spiral tension spring, which slides in a bushing of the cylindrical holder.

19. Use according to one of Claims 15 to 18, **characterized in that** a metering device consists of a round, plane bottom plate into which star-shaped precision grooves are introduced which open into a common central bore, and of a plane, round top plate which is glued thereon and which likewise has such a central bore.

20. Use according to one of Claims 15 to 19, **characterized in that** the metering device has, in each case on the bottom plate and the top plate, a groove for receiving an O-ring seal.

## Revendications

1. Micro-soupape pour l'usage unique pour l'utilisation dans la technique médicale ou la chimie analytique, avec un boîtier avec au moins un siège de soupape et un élément d'obturation fondant sous apport de chaleur pour ouvrir ou obturer la micro-soupape, et avec des éléments électriques chauffants servant à l'apport de chaleur, une ouverture centrale de l'élément d'obturation étant créée en raison de la fusion de l'élément d'obturation fondant pour ouvrir la micro-soupape, ou l'élément d'obturation obstruant l'ouverture centrale lors de la fusion afin d'obturer la micro-soupape.

2. Micro-soupape selon la revendication 1, **caractérisée en ce que** les diaphragmes spéciaux supplémentaires sont disposés au niveau du siège de la soupape et/ou de l'élément d'obturation.

3. Micro-soupape selon la revendication 1 ou 2, **caractérisée en ce que** les éléments d'obturation fondant sous apport de chaleur sont des films, des plaquettes, et/ou des bouchons, fabriqués en des matériaux plastiques ou en cires fondant facilement, et peuvent présenter, le cas échéant, un canal central.

4. Micro-soupape selon l'une quelconque des revendications 1 à 3, **caractérisée en ce que** l'élément électrique chauffant est une résistance ohmique qui peut, le cas échéant, être imprimé ou métallisé, réalisé sous forme de résistance PTC et/ou de filament chauffant et pressé de façon fixe sur les éléments d'obturation ou appliqué directement sur ceux-ci.

5. Micro-soupape selon la revendication 1 ou 2, **caractérisée en ce que** les éléments d'obturation sont faits d'un matériau électriquement conducteur.

6. Micro-soupape selon l'une quelconque des revendications 1 à 5, **caractérisée en ce que** des plaquettes de résistance trouées, de préférence en technique SMD, servent d'éléments électriques chauffants, leurs ouvertures de soupape étant obturées par des éléments d'obturation fondant sous apport de chaleur qui sont imprimés, encollés et/ou soudés.

7. Micro-soupape selon l'une quelconque des revendications 1 à 4, **caractérisée en ce que** les éléments électriques chauffants représentent des résistances PTC trouées, de préférence en forme de plaquette, disquette et en technique SMD, celles-ci représentant des éléments thermostatisés chauffants et des sièges de soupape avec des ouvertures de soupape.

8. Micro-soupape selon la revendication 2, **caractérisée en ce que** les diaphragmes spéciaux sont faits en feutre fibreux, par exemple en cellulose contenant des capillaires fines.

9. Micro-soupape selon la revendication 2, **caractérisée en ce que** les diaphragmes spéciaux sont réalisés sous forme de tamis, trou, grille ou plaquette poreuse et sont faits en métal ou en plastique.

10. Micro-soupape selon l'une quelconque des revendications précédentes, **caractérisée en ce que** les éléments électriques chauffants avec siège de soupape et ouverture de soupape, des résistances SMD trouées ou résistances PTC avec leurs éléments d'obturation appliqués sont encollés ou coulés dans des boîtiers avec des canaux d'admission et de sortie de telle façon qu'ils séparent des canaux d'admission et de sortie comme des cloisons étanches au gaz.

11. Micro-soupape selon l'une quelconque des revendications précédentes, **caractérisée en ce que** les éléments électriques chauffants peuvent être activés par un tel appel de courant, qu'en cas d'utilisation de liquide, une formation de bulles de vapeur se produit au voisinage des éléments chauffants.

12. Micro-soupape selon l'une quelconque des revendications précédentes, **caractérisée en ce qu'**une chambre remplie d'air est disposée avant et/ou à la suite de chaque élément électrique chauffant avec élément d'obturation.

13. Micro-soupape selon l'une quelconque des revendications précédentes, **caractérisée en ce que** l'élément d'obturation est un film d'obturation qui est appliqué, à titre d'exemple imprimé ou métallisé, directement sur les éléments électriques chauffants, ainsi que sur les conduits de raccordement électriques associés et prises de raccordement.

14. Micro-soupape selon l'une quelconque des revendications précédentes, **caractérisée en ce que**, lors du processus de dégagement de la soupape, les éléments électriques chauffants peuvent être chargés électriquement à tel point que leur structure de résistance est destructible, c'est à dire qu'elle peut être interrompue et que ce processus est utilisable pour un déclenchement de signal électrique pour un fonctionnement adéquat de la soupape.

15. Utilisation d'une micro-soupape selon l'une quelconque des revendications 1 à 14 dans un dispositif d'application de médicaments par perfusion, une cassette à usage unique avec des micro-soupapes, des répartiteurs, des raccords luerlock et des dispositifs de dosage étant utilisée, à laquelle sont raccordés des dispositifs de perfusion par gravité, de pompe ou de pression conventionnels via des soupapes unidirectionnelles en association avec des récipients de médicaments, réalisés sous forme de seringue à injection commandées par ressort, et où ces micro-soupapes peuvent être ouvertes par l'unité d'alimentation en courant à commande programmable externe, attachée, à des moments pré-programmés pour vider leur contenu dans les voies de perfusion au patient raccordé.

16. Utilisation selon la revendication 15, **caractérisée en ce que** le dispositif des raccords luerlock, des micro-soupapes, des répartiteurs et éventuellement encore des capillaires de dosage utilisées, est de préférence aménagé en forme d'étoile afin de minimiser la taille constructive et l'espace mort.

17. Utilisation selon la revendication 15 ou 16, **caractérisée en ce que** les seringues d'injection commandées par ressort sont disposées dans un support cylindrique présentant quatre ou plus de seringues cylindriques avec des rainures de seringue respectivement associées avec des éléments de ressort à traction respectivement associés.

18. Utilisation selon la revendication 17, **caractérisée en ce que** l'élément de ressort à traction est composé d'un tuyau de tige de traction avec une plaque de pression poussoir articulée et un ressort à traction en spirale disposé à l'intérieur, qui glisse dans une douille du support cylindrique.

19. Utilisation selon l'une quelconque des revendications 15 à 18, **caractérisée en ce qu'**un dispositif de dosage comprend une plaque de fond ronde, plane, dans laquelle des rainures de précision en forme d'étoile sont aménagées qui débouchent sur un perçage central commun, et une plaque de couverture plane et ronde collée sur la plaque de fond, présentant également un perçage central.

20. Utilisation selon l'une quelconque des revendications 15 à 19, **caractérisée en ce que** le dispositif de dosage présente une rainure pour la réception d'une bague d'étanchéité torique sur la plaque de fond comme sur la plaque de couverture.
